# EUROPEAN PATENT SPECIFICATION

(11) **EP 4 238 570 B1**
(45) Date of publication and mention of the grant of the patent: **10.09.2025**
(21) Application number: 22865878.7
(22) Date of filing: 19.12.2022
(51) Int. Cl.: A61K 31/704, A61P 17/14, A61K 8/63, A61Q 7/00, A61Q 5/02, A61Q 5/06, A61Q 5/12

(54) **HAIR GROWTH PROMOTING COMPOSITION AND MEDICAL USE**
HAARWUCHSFÖRDERNDE ZUSAMMENSETZUNG UND MEDIZINISCHE VERWENDUNG
COMPOSITION FAVORISANT LA CROISSANCE DES CHEVEUX ET UTILISATION MÉDICALE

(30) Priority: 18.01.2022 CN 202210056919; 18.01.2022 CN 202210056920
(43) Date of publication of application: 06.09.2023
(73) Proprietor: Guangxi Xinhai Pharmaceutical Technology Co., Ltd, Liuzhou City, Guangxi 545000 (CN)
(72) Inventor: GAO, Hongwei, Suzhou Jiangsu 215123 (CN); YUAN, Renyikun, Songyuan Jilin 131200 (CN); YANG, Shilin, Beijing 100020 (CN)
(74) Representative: Bandpay & Greuter
(86) International application number: PCT/CN2022/139994
(87) International publication number: WO 2023/138280

(56) References cited:
- CN-A- 108 451 964
- CN-A- 111 249 289
- CN-A- 112 656 802
- CN-A- 113 318 002
- CN-A- 114 288 309
- CN-A- 114 404 430
- KR-B1- 102 213 617
- WANG LIMING, XU QIONGMING, SU SHENG, LIU JIANGYUN, FENG YULIN, LI XIAORAN, ZHU WEIFENG, YANG SHILIN: "Simultaneous Purification of Pulchinenoside B 4 and B 5 from Pulsatilla chinensis Using Macroporous Resin and Preparative High Performance Liquid Chromatography", INDUSTRIAL & ENGINEERING CHEMISTRY RESEARCH, AMERICAN CHEMICAL SOCIETY, vol. 51, no. 45, 14 November 2012 (2012-11-14), pages 14859 - 14866, XP093056365, ISSN: 0888-5885, DOI: 10.1021/ie302165v
- NAM YOU JIN, LEE EUN YOUNG, CHOI EUN‐JU, KANG SANGJIN, KIM JINWAN, CHOI YONG‐SOO, KIM DONG HYUN, AN JI HAE, HAN INBO, LEE SUNGHOU,: "CRH receptor antagonists from Pulsatilla chinensis prevent CRH‐induced premature catagen transition in human hair follicles", JOURNAL OF COSMETIC DERMATOLOGY, BLACKWELL SCIENCE, OXFORD, GB, vol. 19, no. 11, 1 November 2020 (2020-11-01), GB , pages 3058 - 3066, XP093056366, ISSN: 1473-2130, DOI: 10.1111/jocd.13328

## Description

### TECHNICAL FIELD

The present disclosure relates to the technical field of medicine. More specifically, the present disclosure relates to a composition capable of promoting hair growth, a drug capable of promoting hair growth, and application thereof.

### BACKGROUND

Alopecia refers to pathologic hair loss, that is, abnormal or excessive hair loss. Alopecia can be caused by multiple reasons, such as androgen abnormality, psychic trauma, genetic factors, endocrine abnormality, malnutrition, vascular dysfunction, chemical factors, mechanical irritation, developmental defect, seasonal hair loss, etc. Currently, there are minoxidil and finasteride that are approved by FDA to promote hair growth. Although the two drugs can promote hair growth to a certain extent, there are inevitable side effects. Long-term use of minoxidil will lead to edema or arrhythmia, and the efficacy will weaken after 6 months or 1 year. Finasteride can lead to sexual dysfunction, depression or suicidal ideation development, even the increase of probability of birth defects. In addition, the two drugs that can promote hair growth have slow curative effect and high cost in treatment. Therefore, it is currently urgent to develop a composition with non-toxic side effects, rapid curative effect, low cost and effective hair growth promotion.

You Jin Nam MS et al., discloses that Pulsatilla chinensis blocks corticotropin-releasing hormone (CRH) receptor function and that saponins derivatives from P. chinensis could be a pharmaceutical and cosmestic approach to treat stress-induced hair loss (Journal of cosmetic dermatology 2020; 19 (11):3058-3066). **SUMMARY**

An object of the present disclosure is to solve at least above problems, and to provide, at least, the advantages that will be described later. The present invention is defined by the claims. Any subject-matter falling outside the scope of the claims is provided for information only.

An object of the present application is to provide a composition for use in the treatment of alopecia, wherein pulchinenoside B4 or pulchinenoside B5 is as an only active component in the composition, and the compositioncan be made into cream, pills, dripping pills, capsules, granules, powder, paint, cataplasma, sprays, oral liquids, decoction, injections, sustained-release preparations, or controlled-release preparations.

In order to achieve these objects and other advantages according to the present disclosure, a composition capable of promoting hair growth is provided, comprising: a pulchinenoside active component and auxiliaries;
wherein the pulchinenoside active component is pulchinenoside B4 or pulchinenoside B5.

Preferably, wherein pulchinenoside B4 has a molecular formula of C₅₉H₉₆O₂₆, a molecular weight of 1221.39, belongs to lupinane-type pentacyclic triterpenoid saponin, and has a structural formula (I) as follows: 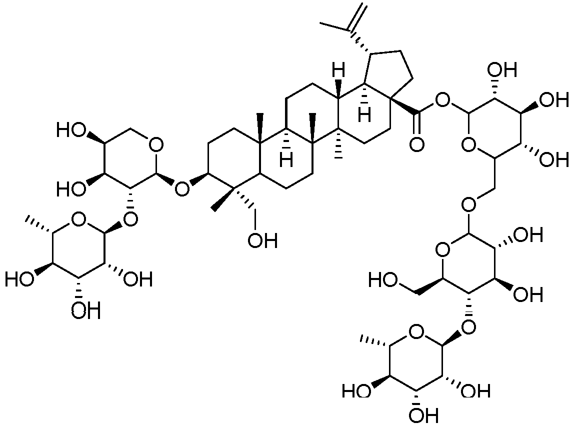 wherein pulchinenoside B5 has a molecular formula of C₅₉H₉₈O₂₆, a molecular weight of 1223.41, and has a structural formula (II) as follows:

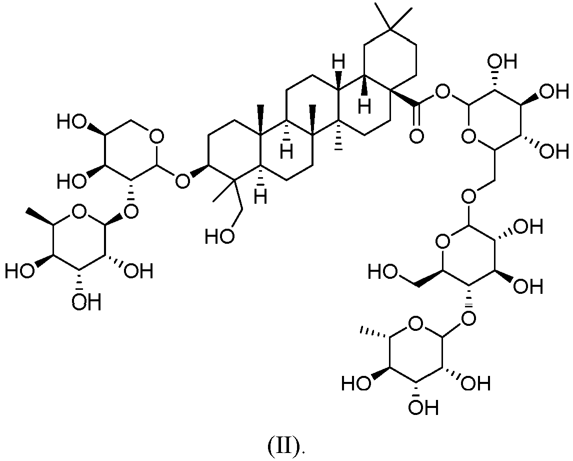

Preferably, an effective concentration of pulchinenoside B5 is 0.5-2 mg/mL.

Preferably, an effective concentration of pulchinenoside B4 is 0.5-2 mg/mL.

Preferably, the composition capable of promoting hair growth can be prepared into shampoo, hair conditioner, hair essential oil, hair essence, hair spray, hair-growth liquid, styling gel, hair tonic, hair mask, hair fixative, mousse, hairdressing gel, or hair dye. Said formulations do not fall within the scope of the claimed subject-matter.

The present disclosure at least comprises the following beneficial effects.
1. The active component of the composition or drug capable of promoting hair growth has good safety and non-toxic side effects.
2. The composition capable of promoting hair growth is used two consecutive weeks to be effective, has rapid curative effect, and short treatment cycle.
3. In view of the current clinical demand for drugs to promote hair growth, a new application of pulchinenoside B4 or pulchinenoside B5 in promotion of hair growth is discovered in the present disclosure.
4. Pulchinenoside B4 or pulchinenoside B5 of the present disclosure has lower price than the existing western medicine, which can reduce the cost of treatment, reduce the economic burden of patients, and meet the current clinical needs.

Other advantages, objects and features of the present disclosure will be partially reflected by the following description, and will be partially understood by those skilled in the art through researching and practicing the present disclosure.

### BRIEF DESCRIPTION OF THE DRAWINGS

FIG. 1 is a skin picture of a hair removal area on the back of mice in each group according to Embodiment 1 of the present disclosure.
FIG. 2 is a diagram for showing the HE staining results of the hair removal area skin of mice in each group according to Embodiment 1 of the present disclosure.
FIG. 3 is a diagram for showing the statistical results of the number of hair follicles of mice in each group according to Embodiment 1 of the present disclosure.
FIG. 4 is a diagram for showing the scores of the hair growth speed of mice in each group according to Embodiment 1 of the present disclosure.
FIG. 5 is a diagram for showing the statistical results of the weight of mice in each group according to Embodiment 1 of the present disclosure.
FIG. 6 is a skin picture of a hair removal area on the back of mice in each group according to Embodiment 2 of the present disclosure.
FIG. 7 is a diagram for showing the HE staining results of the hair removal area skin of mice in each group according to Embodiment 2 of the present disclosure.
FIG. 8 is a diagram for showing the statistical results of the number of hair follicles of mice in each group according to Embodiment 2 of the present disclosure.
FIG. 9 is a diagram for showing the scores of the hair growth speed of mice in each group according to Embodiment 2 of the present disclosure.
FIG. 10 is a diagram for showing the statistical results of the weight of mice in each group according to Embodiment 2 of the present disclosure.

### DETAILED DESCRIPTION

The present disclosure will be further described in detail hereinafter with reference to the accompanying drawings, so that those skilled in the art can implement the present disclosure with reference to the specification.

It should be noted that terms such as "having", "including" and "comprising" as used herein do not exclude presence or addition of one or more other elements or combinations thereof.

It should be noted that the experimental methods described in the following embodiments are all conventional methods unless otherwise specified, and the reagents and materials are commercially available unless otherwise specified.

### Embodiment 1

A composition capable of promoting hair growth adopts pulchinenoside B4 as an active component.

### (1) Experimental animals

The experimental animals are purchased from National Institutes for Food and Drug Control with a production license No. of SCXK2017 (Jing)-0005, KM mice, male, 18-20 g, SPF grade. The experimental animals are raised under a barrier environment in the Experimental Animal Center of the Institute of Basic Theory for Chinese Medicine, China Academy of Chinese Medical Sciences with a production license No. of SYXK (Jing) 2021-0017.

### (2) Experimental reagents

Depilatory: Veet hair removal cream produced by Reckitt Benckiser (France) Co., Ltd, the approval number of imported cosmetics: National cosmetics special import J20130034; positive drug: minoxidil liniment produced by Shanxi Zhendong Ante Biopharmaceutical Co., Ltd.

### (3) Modeling and administration method

Except for a normal control group, the back of all the mice in the other groups are subjected to hair removal.

Animal grouping: a blank group, a model group, a Vaseline group, a pulchinenoside B4-low dose group, a pulchinenoside B4-medium dose group, a pulchinenoside B4-high dose group, a positive drug of minoxidil liniment group, 10 mice per group, pulchinenoside B4 and minoxidil liniment in each group are dissolved with Vaseline. The mice in the Vaseline group are administered Vaseline, the low dose, and medium dose and high dose of pulchinenoside B4 are 0.5, 1, and 2 mg/mL, respectively. The mice are administered twice a day, after 2 weeks of continuous administration, the skin tissue of a hair removal area on the back of the mice in each group is taken, is fixed and is subjected to HE staining, and the number of hair follicles is counted under a microscope.

### (4) Index observation

### (A) Hair growth on the back

Comparation of hair growth in the hair removal areas on the back of mice in each group is carried out by photos to evaluate the speed of hair growth (scoring standard is according to a reference (Tian Xia, Wenqian Li, Xing Xia, Weiping He, Xiaoyun Zhu, The Research on the Promoting Effect of Ginkgo Pilatory Mixture on Hair Growth in Mice, Guangxi Chinese medicine, 2013, 36(05): 69-72.): non-hair growth: 0, short hair grows all over the hair removal area: 1, the length and density of new hair is about half of that of a non-hair removal area: 2, and there is no difference between the new hair growth area and the non-hair removal area: 3).

### (B) The number of hair follicles on the hair removal area

After the mice are sacrificed, the skin tissue of the back is fixed, is embedded with paraffin to obtain a paraffin-embedded section, is subjected to HE staining, and is counted the number of hair follicles to evaluate the effect of hair growth promotion.

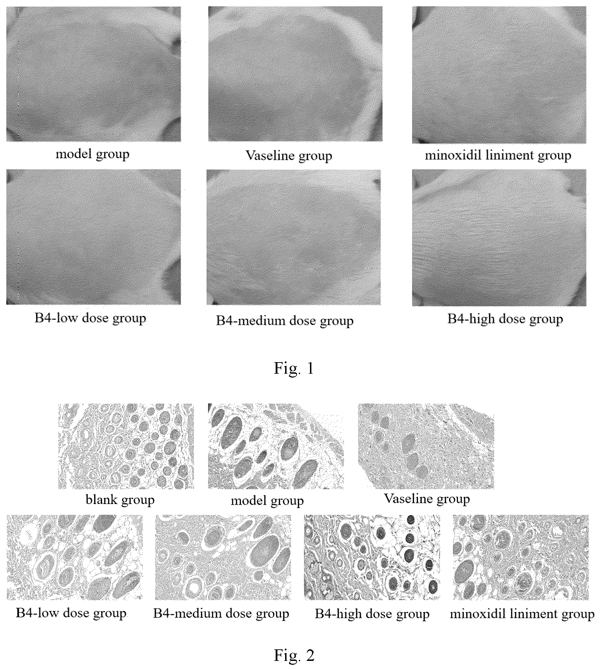

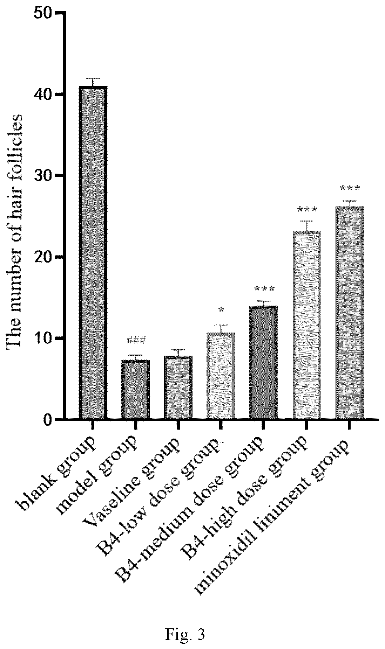

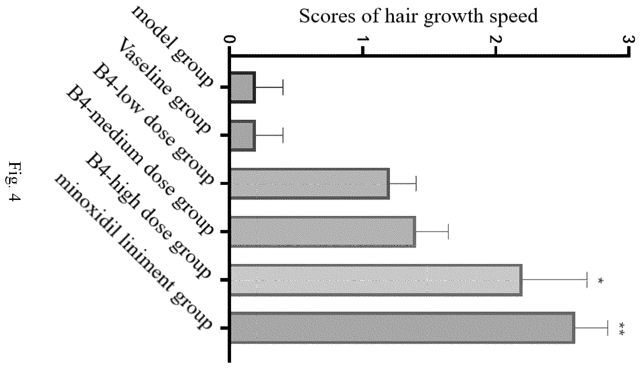

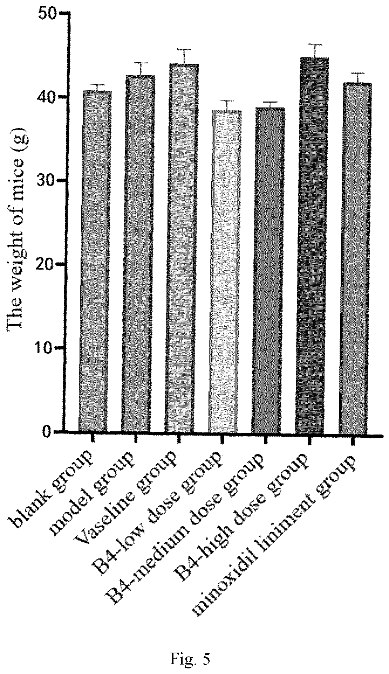

The mice are administered after 2 weeks, and the photos of the skin tissue of the hair removal area on the back of mice in each group is obtained. It can be seen from Fig. 1 that there is less hair growth on the hair removal area on the back of the mice in the model group, and it indicates that the dissolving matrix of Vaseline has no significant effect on hair growth and the dissolving matrix has no promotion effect on hair growth. The low dose of pulchinenoside B4, medium dose of pulchinenoside B4, high dose of pulchinenoside B4, and the positive drug of minoxidil liniment can promote hair growth in the hair removal area on the back of mice in different degree. The skin tissue of the hair removal area on the back of the mice in each group is obtained, is embedded, and is stained with HE. The HE staining result is shown as Fig. 2, and it can be seen from Fig. 2 that the number of hair follicles of the mice in the pulchinenoside B4-low dose group, the pulchinenoside B4-medium dose group, and the pulchinenoside B4-high dose group is significantly increased compared with the model group. The number of hair follicles in the skin tissue of the mice in each group is counted according to the HE staining photos, and the result is shown as Fig. 3. Compared with the blank group, the number of hair follicles in the skin tissue of the hair removal area on the back of mice in the model group is significantly reduced. The matrix of Vaseline has no significant effect on hair growth. The low dose of pulchinenoside B4, medium dose of pulchinenoside B4, and high dose of pulchinenoside B4 can significantly increase the number of hair follicles in the skin tissue of the hair removal area on the back of mice. The results in Fig. 2 and Fig. 3 show that pulchinenoside B4 can increase the number of hair follicles. The scores of hair growth speed of mice in each group are shown as Fig. 4, it can be seen from Fig. 4 that there is no significant difference in the scores of hair growth speed of mice between the Vaseline group and the model group, and it indicates that the dissolving matrix has no significant effect on hair growth. Compared with the model group, the hair growth speed of the hair removal area on the back of mice in the pulchinenoside B4-high dose group is significantly increased. In addition, the result of Fig. 5 shows that there is no significant effect on the weight of mice after administration for 2 weeks, and it indicates that pulchinenoside B4 has non-toxic side effects.

In conclusion, pulchinenoside B4 has the following technical effects:
(1) promoting hair growth of the hair removal area of the mice.
(2) improving the hair growth speed.
(3) increasing the number of hair follicles.
(4) non-toxic side effects.
(5) having instant effect after continuous administration for 2 weeks, and having rapid curative effect and short treatment cycle.

The present disclosure may include multiple examples according to the component type, component content, dosage form, etc., such as:
Example 1: cleaning and care products with pulchinenoside B4 as an active component.

Pulchinenoside B4 as active component can be prepared into shampoo, hair conditioner, hair essential oil, hair essence, hair spray, hair-growth liquid, styling gel, hair tonic, hair mask, hair fixative, mousse, hairdressing gel, or hair dye.

Example 2: emulsion or cream with pulchinenoside B4 as an active component.

Semi solid state at room temperature is usually described cream, and fluid state is described milky or emulsion. Emulsion is a system formed by two immiscible liquids, one phase of which is dispersed in the other phase as tiny droplets, wherein the phase in the form of droplets is usually called dispersed phase or internal phase, and the other phase is called disperse medium or external phase.

Frequently-used auxiliaries of emulsion or cream include: emulsifiers, antioxidants, tackifiers, pH regulators, oily raw materials.

The emulsifier is necessary to form stable emulsion.

The pH regulator is used to avoid excessive irritation and improve stability, and a buffer solution is commonly used to stabilize a pH value of a drug solution, wherein the buffer solution includes boric acid buffer solution, phosphate buffer solution, etc.

Methyl cellulose (MC), polyvinyl alcohol (PVA), Carbopol, polyvinyl pyrrolidone (PVP), etc. are commonly used as the tackifier, and CMC-Na is not commonly used because it is incompatible with alkaloids and chlorhexidine.

Oily raw materials are commonly added into emulsion or cream, which plays a role in shaping, stabilizing or imparting color, odor and other characteristics. The amount of the oily raw materials in a formula is low, but they are extremely important, and the oily raw materials mainly include lipids, such as waxes, hydrocarbons and synthesize lipids.

Example 3: oral liquid with pulchinenoside B4 as an active component.

Take the oral liquid as an example, oral liquid is a new dosage form developed based on decoction and injections, which has the advantages of small dosage, rapid absorption, stable quality, convenient carrying and taking, and easy storage, contains multiple components, and has a significant impact on the quality and taste. How to maximize the retention of active components and improve the taste is a difficult problem in the selection of auxiliaries without changing the structure and function of the main active components. The addition of auxiliaries in oral liquid can improve the taste, improve clarity, enhance the stability and improve the product quality.

Common auxiliaries of oral liquid include: solvents, fragrances, flavoring agents, clarifying agents, preservatives, etc. These auxiliaries can be simultaneously added, or one of them is added. Wherein the solvents need to be added, and water can be used as a solvent. Different combinations of the auxiliaries include: sweeteners, fragrances, clarifying agents or preservatives, or a combination of sweeteners and preservatives, it is preferred that the auxiliaries include the combination of sweeteners and preservatives. Some auxiliaries have the functions of both sweetness and fragrance enhancement, and only one auxiliary now needs to be added.

In view of the oral liquid, it is preferred that the sweetener is selected from one or more of protein glucose, xylitol, aspartame and sucralose.

In view of the oral liquid, it is preferred that the preservative is selected from one or more of paraben, butyl hydroxyanisole, 2,6-di-tert-butyl-4-methylphenol and sorbic acid.

The preservative may be paraben, 2,6-di-tert-butyl-4-methylphenol or sorbic acid, preferably 2,6-di-tert-butyl-4-methylphenol, and the preservative may be also a combination of paraben and 2,6-di-tert-butyl-4-methylphenol, a combination of 2,6-di-tert-butyl-4-methylphenol and sorbic acid, a combination of paraben and sorbic acid, or a combination of paraben, 2,6-di-tert-butyl-4-methylphenol and sorbic acid.

In view of the oral liquid, it is preferred that the fragrance may be fruit essence.

In view of the oral liquid, it is preferred that the clarifying agent may be is one or a mixture of chitosan and gelatin.

Example 4: tablets with pulchinenoside B4 as an active component.

The tablets have the advantageous effects of accurate dosage, stable quality, and convenient taking, carrying and transportation.

In view of the tablets, the auxiliaries include one or more of diluents, adhesives, lubricants and disintegrants, it is preferred that a combination of the diluent, the adhesive, the lubricant and the disintegrant.

In view of the tablets, it is preferred that the diluent is one or more of celluloses and inorganic salts, such as microcrystalline cellulose, calcium sulfate, calcium monophosphate, medical calcium carbonate, mannitol, etc., and the diluent is used to increase the volume of raw materials and to facilitate shaping.

In view of the tablets, it is preferred that the adhesive is one or more of water, ethanol, sodium carboxymethyl cellulose, hydroxypropyl cellulose, methyl cellulose, ethyl cellulose, gelatin and polyvinylpyrrolidone.

In view of the tablets, it is preferred that the lubricant is one or more of magnesium stearate, micro-silica gel, talc powder, hydrogenated vegetable oil, polyethylene glycol and magnesium lauryl sulfate.

In view of the tablets, it is preferred that the disintegrant is one or more of low-substituted hydroxypropyl cellulose, polyvinylpolypyrrolidone and croscarmellose sodium.

Example 5, capsules with pulchinenoside B4 as an active component.

In the present disclosure, the capsules mainly improve stability and bioavailability, and the auxiliaries are capsule shells that may be hard capsule shells or soft capsule shells.

Example 6, granules with pulchinenoside B4 as an active component.

The granules can be swallowed directly or taken with warm water, and have the advantages of convenient applying and carrying, rapid dissolution and absorption. The auxiliaries of the granules are similar with those of the tablets, relates to one or more of fillers, adhesives, wetting agents, disintegrants, lubricants and film coating materials.

In view of the granules, it is preferred that the filler is one or more of celluloses and inorganic salts, such as microcrystalline cellulose, calcium sulfate, calcium monophosphate, medical calcium carbonate, mannitol, etc., and the filler is used to increase the volume of raw materials and to facilitate shaping.

In view of the granules, it is preferred that the adhesive is one or more of water, ethanol, sodium carboxymethyl cellulose, hydroxypropyl cellulose, methyl cellulose, ethyl cellulose, gelatin and polyvinylpyrrolidone.

In view of the granules, it is preferred that the wetting agent is water or ethanol, or a mixture of both.

In view of the granules, it is preferred that the disintegrant is one or more of low-substituted hydroxypropyl cellulose, polyvinylpolypyrrolidone and croscarmellose sodium

In view of the granules, it is preferred that the lubricant is one or more of magnesium stearate, micro-silica gel, talc powder, hydrogenated vegetable oil, polyethylene glycol and magnesium lauryl sulfate.

In view of the granules, it is preferred that the film coating materials are one or more of hydroxypropyl methylcellulose, polyethylene glycol, cellulose acetate phthalate and polyvinyl acetal diethylamino acetate.

Example 7, powder with pulchinenoside B4 as an active component.

The powder may be prepared in the present disclosure, and the powder is readily for dosage distribution and taking.

Take beverages as an example, the composition of the present disclosure may be prepared into beverages with vary flavor, and will be very popular as a daily beverage.

The auxiliaries of the beverages include at least one of the clarifiers, preservatives and fragrances.

The composition of the present disclosure may also be made into other powder, such as functional milk powder that contains main auxiliaries of milk powder, such as defatted milk powder, defatted sugar-free milk powder.

The dosage in per unit product of the above multiple examples can be adjusted to adapt to different application, such as drugs, health products, food, etc.

### Embodiment 2

A composition capable of promoting hair growth adopts pulchinenoside B5 as an active component.

### (1) Experimental animal

The experimental animals are purchased from National Institutes for Food and Drug Control with the production license No. of SCXK2017 (Jing)-0005, KM mice, male, 18-20 g, spf grade. The experimental animals are raised under a barrier environment in the Experimental Animal Center of the Institute of Basic Theory for Chinese Medicine, China Academy of Chinese Medical Sciences with the production license No. of SYXK (Jing) 2021-0017.

### (2) Experimental reagents

Depilatory: Veet hair removal cream produced by Reckitt Benckiser (France) Co., Ltd. Approval number of imported cosmetics: National cosmetics special import J20130034; positive drug: minoxidil liniment produced by Shanxi Zhendong Ante Biopharmaceutical Co., Ltd.

### (3) Modeling and administration method

Except for the normal control group, the back of all the mice in the other groups are subjected to hair removal.

Animal grouping: a blank group, a model group, a Vaseline group, a pulchinenoside B5-low dose group, a pulchinenoside B5-medium dose group, a pulchinenoside B5-high dose group, a positive drug of minoxidil liniment group, 10 mice per group, pulchinenoside B5 and minoxidil liniment in each group are dissolved with Vaseline. The mice in the Vaseline group are administered Vaseline, low dose, and medium dose and high dose of pulchinenoside B5 are 0.5, 1, and 2 mg/mL, respectively. The mice are administered twice a day, after 2 weeks of continuous administration, the skin tissue of a hair removal area on the back of the mice in each group is obtained, is fixed and is subjected to HE staining, and the number of hair follicles is counted under a microscope.

### (4) Index observation

### (A) Hair growth on the back

Comparation of hair growth in the hair removal areas on the back of mice in each group is carried out by photos to evaluate the speed of hair growth (scoring standard is according to a reference (Tian Xia, Wenqian Li, Xing Xia, Weiping He, Xiaoyun Zhu, The Research on the Promoting Effect of Ginkgo Pilatory Mixture on Hair Growth in Mice, Guangxi Chinese medicine, 2013, 36(05): 69-72.): non-hair growth: 0, short hair grows all over the hair removal area: 1, the length and density of new hair is about half of that of non-hair removal area: 2, and there is no difference between the new hair growth area and the non-hair removal area: 3).

### (B) The number of hair follicles on the hair removal area

After the mice are sacrificed, the skin tissue of the back is fixed, is embedded with paraffin to obtain a paraffin-embedded section, is subjected to HE staining, and is counted the number of hair follicles to evaluate the effect of hair growth promotion.

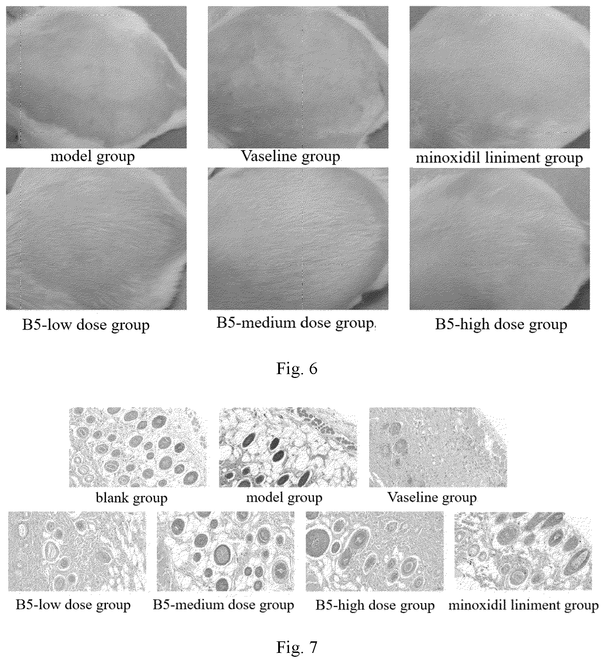

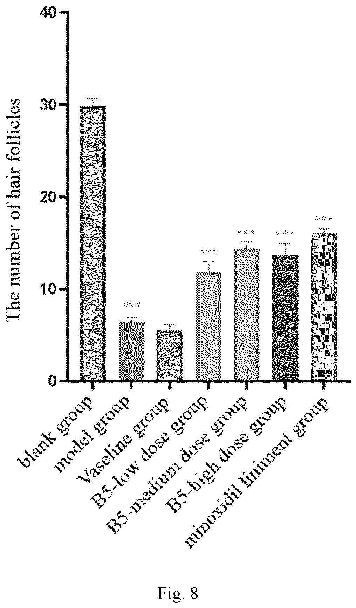

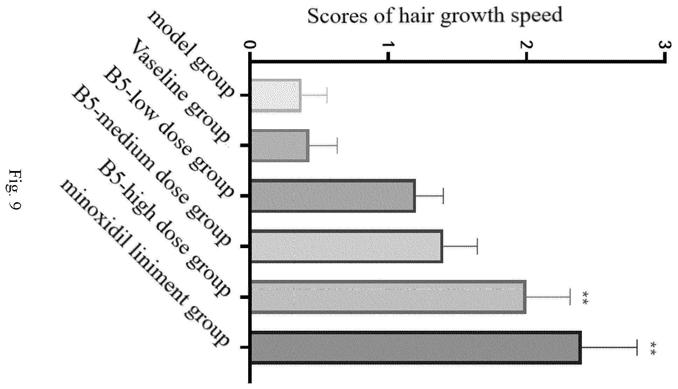

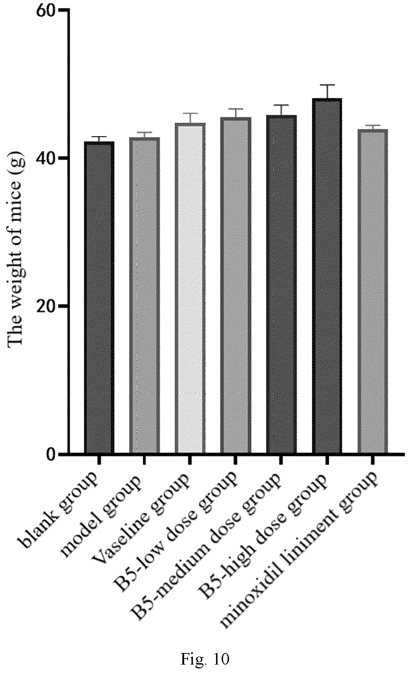

The mice are administered after 2 weeks, and the photos of the skin tissue of the hair removal area on the back of mice in each group is obtained. It can be seen from Fig. 6 that there is less hair growth on the hair removal area on the back of the mice in the model group, and it indicates that the dissolving matrix of Vaseline has no significant effect on hair growth and the dissolving matrix has no promotion effect on hair growth. The low dose of pulchinenoside B5, medium dose of pulchinenoside B5, high dose of pulchinenoside B5, and the positive drug of minoxidil liniment can promote hair growth in the hair removal area on the back of mice in different degree. The skin tissue of the hair removal area on the back of the mice in each group is obtained, is embedded, and is stained with HE. The HE staining result is shown as Fig. 7, and it can be seen from Fig. 7 that the number of hair follicles of the mice in the pulchinenoside B5-low dose group, the pulchinenoside B5-medium dose group, and the pulchinenoside B5-high dose group is significantly increased compared with the model group. The number of hair follicles in the skin tissue of the mice in each group is counted according to the HE staining photos, and the result is shown as Fig. 8. Compared with the blank group, the number of hair follicles in the skin tissue of the hair removal area on the back of mice in the model group is significantly reduced. The matrix of Vaseline has no significant effect on hair growth. The low dose of pulchinenoside B5, medium dose of pulchinenoside B5, and high dose of pulchinenoside B5 can significantly increase the number of hair follicles in the skin tissue of the hair removal area on the back of mice. The results in Fig. 7 and Fig. 8 show that pulchinenoside B5 can increase the number of hair follicles. The scores of hair growth speed of mice in each group are shown as Fig. 9, it can be seen from Fig. 9 that there is no significant difference in the scores of hair growth speed of mice between the Vaseline group and the model group, and it indicates that the dissolving matrix has no significant effect on hair growth. Compared with the model group, the hair growth speed of the hair removal area on the back of mice in the pulchinenoside B5-high dose group is significantly increased. In addition, the result of Fig. 10 shows that there is no significant effect on the weight of mice after administration for 2 weeks, and it indicates that pulchinenoside B5 has non-toxic side effects.

In conclusion, pulchinenoside B5 has the following technical effects:
(1) promoting hair growth of the hair removal area of the mice.
(2) improving the hair growth speed.
(3) increasing the number of hair follicles.
(4) non-toxic side effects.
(5) having instant effect after continuous administration for 2 weeks, and having rapid curative effect and short treatment cycle.

The present disclosure may include multiple examples according to the component type, component content, dosage form, etc., such as:
Example 1: cleaning and care products with pulchinenoside B5 as an active component.

Pulchinenoside B5 as active component can be prepared into shampoo, hair conditioner, hair essential oil, hair essence, hair spray, hair-growth liquid, styling gel, hair tonic, hair mask, hair fixative, mousse, hairdressing gel, or hair dye.

Example 2: emulsion or cream with pulchinenoside B5 as an active component. Semi solid state at room temperature is usually described cream, and fluid state is described milky or emulsion. Emulsion is a system formed by two immiscible liquids, one phase of which is dispersed in the other phase as tiny droplets, wherein the phase in the form of droplets is usually called dispersed phase or internal phase, and the other phase is called disperse medium or external phase.

Frequently-used auxiliaries of emulsion or cream include: emulsifier, antioxidant, tackifier, pH regulator, oily raw materials.

The emulsifier is necessary to form stable emulsion.

The pH regulator is used to avoid excessive irritation and improve stability, and a buffer solution is commonly used to stabilize a pH value of a drug solution, wherein buffer solution includes boric acid buffer solution, phosphate buffer solution, etc.

Methyl cellulose (MC), polyvinyl alcohol (PVA), Carbopol, polyvinyl pyrrolidone (PVP), etc. are commonly used as the tackifier, and CMC-Na is not commonly used because it is incompatible with alkaloids and chlorhexidine.

Oily raw materials are commonly added into emulsion or cream, which plays a role in shaping, stabilizing or imparting color, odor and other characteristics. The amount of the oily raw materials in a formula is low, but they are extremely important, and the oily raw materials mainly include lipids, such as waxes, hydrocarbons and synthesize lipids.

Example 3: oral liquid with pulchinenoside B5 as an active component.

Take the oral liquid as an example, the oral liquid is a new dosage form developed based on decoction and injections, which has the advantages of small dosage, rapid absorption, stable quality, convenient carrying and taking, and easy storage, contains multiple components, and has a significant impact on the quality and taste. How to maximize the retention of active components and improve the taste is a difficult problem in the selection of auxiliaries without changing the structure and function of the main active components. The addition of auxiliaries in oral liquid can improve the taste, improve clarity, enhance stability and improve product quality.

Common auxiliaries of the oral liquid include: solvents, fragrances, flavoring agents, clarifying agents, preservatives, etc. These auxiliaries can be simultaneously added, or one of them is added. Wherein the solvents need to be added, and water can be used as a solvent. Different combinations of the auxiliaries include: sweeteners, fragrances, clarifying agents or preservatives, or a combination of sweeteners and preservatives, it is preferred that the auxiliaries include the combination of sweeteners and preservatives. Some auxiliaries have the functions of both sweetness and fragrance enhancement, and only one auxiliary now needs to be added.

In view of the oral liquid, it is preferred that the sweetener is selected from one or more of protein glucose, xylitol, aspartame and sucralose.

In view of the oral liquid, it is preferred that the preservative is selected from one or more of paraben, butyl hydroxyanisole, 2,6-di-tert-butyl-4-methylphenol and sorbic acid.

The preservative may be paraben, 2,6-di-tert-butyl-4-methylphenol or sorbic acid, preferably 2,6-di-tert-butyl-4-methylphenol, and the preservative may be also a combination of paraben and 2,6-di-tert-butyl-4-methylphenol, a combination of 2,6-di-tert-butyl-4-methylphenol and sorbic acid, a combination of paraben and sorbic acid, or a combination of paraben, 2,6-di-tert-butyl-4-methylphenol and sorbic acid.

In view of the oral liquid, it is preferred that the fragrance may be fruit essence.

In view of the oral liquid, it is preferred that the clarifying agent may be is one or a mixture of chitosan and gelatin.

Example 4: tablets with pulchinenoside B5 as an active component.

The tablets have the advantageous effects of accurate dosage, stable quality, and convenient taking, carrying and transportation.

In view of the tablets, the auxiliaries include one or more of diluents, adhesives, lubricants and disintegrants, it is preferred that the auxiliary is a combination of a diluent, an adhesive, a lubricant and a disintegrant.

In view of the tablets, it is preferred that the diluent is one or more of celluloses and inorganic salts, such as microcrystalline cellulose, calcium sulfate, calcium monophosphate, medical calcium carbonate, mannitol, etc., and the diluent is used to increase the volume of raw materials and to facilitate shaping.

In view of the tablets, it is preferred that the adhesive is one or more of water, ethanol, sodium carboxymethyl cellulose, hydroxypropyl cellulose, methyl cellulose, ethyl cellulose, gelatin and polyvinylpyrrolidone.

In view of the tablets, it is preferred that the lubricant is one or more of magnesium stearate, micro-silica gel, talc powder, hydrogenated vegetable oil, polyethylene glycol and magnesium lauryl sulfate.

In view of the tablets, it is preferred that the disintegrant is one or more of low-substituted hydroxypropyl cellulose, polyvinylpolypyrrolidone and croscarmellose sodium.

Example 5, capsules with pulchinenoside B5 as an active component.

In the present disclosure, the capsules mainly improve stability and bioavailability, and the auxiliaries are capsule shells that may be hard capsule shells or soft capsule shells.

Example 6, granules with pulchinenoside B5 as an active component.

The granules can be swallowed directly or taken with warm water, and have the advantages of convenient applying and carrying, rapid dissolution and absorption. The auxiliaries of the granules are similar with those of the tablets, relates to one or more of fillers, adhesives, wetting agents, disintegrants, lubricants and film coating materials.

In view of the granules, it is preferred that the filler is one or more of celluloses and inorganic salts, such as microcrystalline cellulose, calcium sulfate, calcium monophosphate, medical calcium carbonate, mannitol, etc., and the filler is used to increase the volume of raw materials and to facilitate shaping.

In view of the granules, it is preferred that the adhesive is one or more of water, ethanol, sodium carboxymethyl cellulose, hydroxypropyl cellulose, methyl cellulose, ethyl cellulose, gelatin and polyvinylpyrrolidone.

In view of the granules, it is preferred that the wetting agent is water or ethanol, or a mixture of both.

In view of the granules, it is preferred that the disintegrant is one or more of low-substituted hydroxypropyl cellulose, polyvinylpolypyrrolidone and croscarmellose sodium

In view of the granules, it is preferred that the lubricant is one or more of magnesium stearate, micro-silica gel, talc powder, hydrogenated vegetable oil, polyethylene glycol and magnesium lauryl sulfate.

In view of the granules, it is preferred that the film coating materials are one or more of hydroxypropyl methylcellulose, polyethylene glycol, cellulose acetate phthalate and polyvinyl acetal diethylamino acetate.

Example 7, powder with pulchinenoside B5 as an active component.

The powder may be prepared in the present disclosure, and the powder is readily for dosage distribution and taking.

Take beverages as an example, the composition of the present disclosure may be prepared into beverages with vary flavor, and will be very popular as a daily beverage.

The auxiliaries of the beverages include at least one of the clarifiers, preservatives and fragrances.

The composition of the present disclosure may also be made into other powder, such as functional milk powder that contains main auxiliaries of milk powder, such as defatted milk powder, defatted sugar-free milk powder.

The dosage in per unit product of the above multiple examples can be adjusted to adapt to different application, such as drugs, health products, food, etc.

In conclusion from the above embodiments, the present disclosure discloses a composition capable of promoting hair growth with pulchinenoside B4 or pulchinenoside B5 as the active component. The composition includes pulchinenoside B4 or pulchinenoside B5, and auxiliaries. According to the experiments, it is showed that pulchinenoside B4 and pulchinenoside B5 can promote hair growth in the hair removal area of mice, improve the hair growth speed, increase the number of hair follicles, and has non-toxic side effects, low cost and rapid curative effect. Therefore, pulchinenoside B4 or pulchinenoside B5 and auxiliaries can be prepared into the composition capable of promoting hair growth or drugs capable of promoting hair growth.

## Claims

1. A composition for use in the treatment of alopecia, wherein pulchinenoside B4 or pulchinenoside B5 is as an only active component in the composition, and the composition can be made into cream, pills, dripping pills, capsules, granules, powder, paint, cataplasma, sprays, oral liquids, decoction, injections, sustained-release preparations, or controlled-release preparations.

2. The composition for use in the treatment of alopecia as claimed in claim 1, wherein an effective concentration of pulchinenoside B5 is 0.5-2 mg/mL.

3. The composition for use in the treatment of alopecia as claimed in claim 1, wherein an effective concentration of pulchinenoside B4 is 0.5-2 mg/mL.

## Patentansprüche

1. Zusammensetzung zur Verwendung bei der Behandlung von Alopezie, wobei Pulchinenosid B4 oder Pulchinenosid B5 als einzige Wirkkomponente in der Zusammensetzung vorhanden ist, und die Zusammensetzung zu Creme, Pillen, Tropfenpillen, Kapseln, Granulaten, Pulver, Aufstreichzubereitung, Wickel, Sprays, oralen Flüssigkeiten, Sud, Injektionen, Zubereitungen mit anhaltender Wirkstofffreisetzung oder Zubereitungen mit kontrollierter Wirkstofffreisetzung hergestellt werden kann.

2. Zusammensetzung zur Verwendung bei der Behandlung von Alopezie nach Anspruch 1, wobei eine wirksame Konzentration von Pulchinenosid B5 0,5 bis 2 mg/ml beträgt.

3. Zusammensetzung zur Verwendung bei der Behandlung von Alopezie nach Anspruch 1, wobei eine wirksame Konzentration von Pulchinenosid B4 0,5 bis 2 mg/ml beträgt.

## Revendications

1. Composition pour une utilisation dans le traitement de l'alopécie, dans laquelle le pulchinénoside B4 ou le pulchinénoside B5 est présent à titre d'unique principe actif dans la composition, et laquelle composition peut être mise sous forme de crème, pilules, pilules formées par solidification goutte à goutte, capsules, granules, poudre, préparation topique à appliquer, cataplasme, sprays, liquides à usage oral, décoction, préparations injectables, préparations à libération prolongée, ou préparations à libération contrôlée.

2. Composition pour une utilisation dans le traitement de l'alopécie selon la revendication 1, dans laquelle la concentration efficace de pulchinénoside B5 est de 0,5 à 2 mg/ml.

3. Composition pour une utilisation dans le traitement de l'alopécie selon la revendication 1, dans laquelle la concentration efficace de pulchinénoside B4 est de 0,5 à 2 mg/ml.
